Europäisches Patentamt

European Patent Office

Office européen des brevets

(19)

(11) Publication number: **0 123 443**

**A2**

(12)

# EUROPEAN PATENT APPLICATION

(21) Application number: **84302012.4**

(22) Date of filing: **26.03.84**

(51) Int. Cl.³: **G 01 N 33/52**

(30) Priority: **24.03.83 JP 49306/83**

(43) Date of publication of application:
**31.10.84 Bulletin 84/44**

(84) Designated Contracting States:
**DE GB**

(71) Applicant: **FUJI PHOTO FILM CO., LTD.**
**210 Nakanuma Minami Ashigara-shi**
**Kanagawa 250-01(JP)**

(72) Inventor: **Iwata, Yuzo**
**c/o Fuji Photo Film Co., Ltd 3-11-46 Senzui**
**Asaka-shi Saitama(JP)**

(72) Inventor: **Kitajima, Masao**
**c/o Fuji Photo Film Co., Ltd 3-11-46 Senzui**
**Asaka-shi Saitama(JP)**

(72) Inventor: **Kondo, Asaji**
**c/o Fuji Photo Film Co., Ltd 3-11-46 Senzui**
**Asaka-shi Saitama(JP)**

(74) Representative: **Arthur, Bryan Edward et al,**
**Withers & Rogers 4 Dyer's Buildings Holborn**
**London EC1N 2JT(GB)**

(54) Method of analyzing whole blood sample.

(57) A method of quantitative analysis of an analyte contained in a whole blood sample using a multilayer analytical element comprising a transparent support, at least one reagent layer and a porous spreading layer capable of supplying a liquid sample spotted on the surface of said spreading layer to said reagent layer keeping a substantially constant volume per unit area, the method comprising spotting the whole blood sample on the porous spreading layer:

which is characterized in that the whole blood sample is a blood sample prepared by dilution of a whole blood with an aqueous diluent in a specified volume ratio.

**FIG.1 A**

EP 0 123 443 A2

# METHOD OF ANALYZING WHOLE BLOOD SAMPLE

## BACKGROUND OF THE INVENTION

### Field of the invention

The present invention relates to a method of quantitatively analyzing a substance to be analyzed (analyte) contained in a whole blood by using a mulitilayer analytical element.

### Description of prior arts

Multilayer analytical elements having at least one reagent layer and a porous spreading layer in this order on a transparent support and methods of quantitatively analyzing aqueous liquid samples by using the same, are disclosed in Japanese Patent Publication No. 49(1974)-53888, Jpanese Patent Provisional Publications Nos. 50(1975)-137192, 51(1976)-140191, 52(1977)-3488, 53(1978)-131089, 54(1979)-101398, 55(1980)-90859, 55(1980)-164356, 56(1981)-24576, etc., H. G. Curme & R. W. Spayd, American Clinical Chemical Society Magazine "Clinical Chemistry", vol. 24, pp 1335-1350(1978), etc. There is also described that undiluted serum and blood plasma as well as undiluted whole blood as such can be used as a sample.

Trials in clinical tests for quantitatively analyzing blood sugar with real time by using undiluted whole blood as a sample and a multilayer analytical element as an analytical means are reported in the following literatures; Okubo et al., "Clinical Chemistry", vol. 27, pp 1287-1296 (1981); Ota et al., "Clinical Test Means and Reagent", vol. 5, pp 867-870 (1982); Kobayashi et al, "Fundamental and Clinical", vol. 16, pp 484-486 (1982), etc.

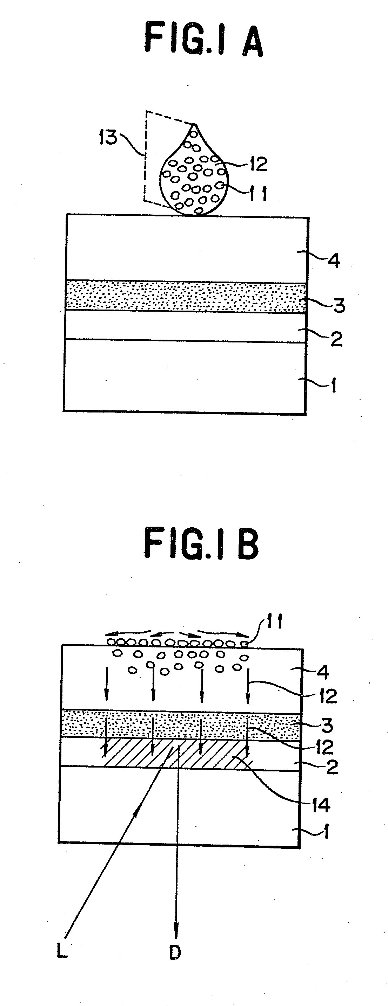

Figure 1 shows diagrammatically one example of a method of analyzing a whole blood sample by the use of the above-mentioned multilayer analytical element. The element is in the form of a multilayer analytical film having a structure wherein a reagent layer 2, a light-reflecting layer 3 and a porous spreading layer 4 are provided on one side of a transparent support 1 (if necessary, functional layers such as a plurality of reagent layers, a barrier layer, a scavenger layer, a buffer layer, a detector, etc. may be inserted between the support and the spreading layer). When a whole blood sample 13 consisting essentially of a material component 11 and a liquid component 12 is spotted on the porous spreading layer 4 of the multilayer analytical film, the whole blood is rapidly spread on the spreading layer in a circular form with an area proportional to the spotted volume, and then the liquid component passes through the light-reflecting layer 3 into the reagent layer 2, while the material component is filtered thereon. Generally, a selective color-forming reagent capable of selectively reacting with an analyte contained in blood is previously incorporated in the reagent layer so that a color-forming reaction takes place in proportion to the amount of the analyte. The amount of the analyte in blood can be determined through colorimetry after photometrically measuring the color optical density of the color-formed area 14 from the support side.

The porous spreading layer can be made of a non-fibrous isotropic porous material such as a membrane filter, a porous material comprising powdery material, a fabric or a paper showing little chromatographic effect, and has such properties that when an aqueous liquid sample is applied (e.g., spotted or droped) thereon, the sample radially spreads in horizontal direction and then permeates through the layer vertically, supplying said liquid and analyte to the reagent layer positioned below the spreading layer keeping a substantially constant volume and amount per unit area, respectively. This effect is called a spreading effect or a metering effect. Particularly, when a fabric stated in Japanese Patent Provisional Publication Nos. 55(1980)-164356 and 57(1982)-66359 or a continuous void-containing fine powder structure disclosed in Japanese Patent Provisional Publication No. 55(1980)-90859 is used as a material of the porous spreading layer, it shows a spreading effect on blood plasma and serum as well as a sample such as whole blood containing the material component, and accordingly quantitative analysis using whole blood as the sample is possible.

The present inventors have examined in detail the the aforementioned test results of the clinical trial for the analysis of whole blood sample using the multilayer analytical element, made confirmative experiments, and found that when a fresh whole blood wherein hematocrit value, fluidity, etc. are approximately within the normal range (hereinafter referred to as normal whole blood) is used as the test sample, the above-mentioned analytical method gives satisfactory analytical result, but when a whole blood wherein the amount of the analyte and hematocrit value are abnormally high or fluidity is poor or which is kept for several hours or longer (hereinafter referred to as abnormal whole blood) is used as the test sample, the analytical performances such as accuracy, precision, etc. sometimes remarkably lowers.

It is highly desired to obtain analytical results on a chemical component (analyte) contained in a blood simply and quickly by using a whole blood sample, that is, a collected blood having been subjected to no treatment, and employing dry sheet-type or film-type analytical means. Analytical methods and elements using whole blood as a sample are disclosed in U. S. Patent Nos. 3,298,789 and 3,395,082, etc., in addition to the specifications of the aforementioned Japanese Patent Publication, Japanese Patent Provisional Publications and literatures. However, such sheet-type analytical means as possessing characteristics mentioned above is considered that analytical performance is insufficient as compared with that of the conventional wet-process analytical method using serum. The reason is thought as follows: the analytical performance depends on the properties of a whole blood to be used as the sample, that is, when an abnormal whole blood is used as the sample, the analytical performance lowers, as compared with the case where a normal whole blood is used as the sample, and therefore, the use of a whole blood sample inherently under many restrictions. Hence, the enlargement of tolerance for the sample is an important factor to increase the analytical performance of the sheet-type analytical means to a level equal to that of the conventional wet process.

## SUMMARY OF THE INVENTION

The present inventors have discovered that if a whole blood diluted to a certain volume under such conditions that blood does not hemolyze is employed as the test sample in place of a whole blood as such, satisfactory analytical results can be obtained on a normal whole blood as well as an abnormal whole blood even though the same dry analytical sheet is used. The present invention is based on this discovery.

The reason why the tolerance for the sample in the dry analytical sheet method is enlarged by the use of a diluted whole blood is not clear. However, the reason is thought that the concentration of materials acting as interfering substances is relatively lowered.

An object of the present invention is to provide an improved analytical method employed for analyzing an analyte contained in a whole blood sample by the use of a multilayer analytical element.

Another object of the present invention is to provide a method capable of exhibiting good analytical performance even when an abnormal whole blood is used as a test sample, said performance being substantially equal to that in the case where a normal whole blood is used as the test sample.

Still another object of the present invention is to provide a method capable of increasing the analytical performance using the multilayer analytical element to a level of the conventional wet process, maintaining the advantageous aspects of the method using multilayer analytical, namely, by simplicity and quickness.

The present invention provides a method of quantitatively analyzing an analyte contained in a whole blood sample by the use of a multilayer analytical element having at least one reagent layer provided on one side of a light-transmissive water-impermeable support and a porous spreading layer forming the outermost layer of said element and being able to supply a liquid sample applied on the surface of said spreading layer to said reagent layer keeping a substantially constant volume per unit area, the method comprising applying the whole blood sample on the porous spreading layer:

which is characterized in that the whole blood sample is a blood sample prepared by dilution of a whole blood with an aqueous diluent in a specified volume ratio.

## BRIEF DESCRIPTION OF DRAWINGS

Figure 1 shows diagramatically one embodiment of the method of analysis of a whole blood sample using a multilayer analytical film, wherein Figure 1A shows spotting of the whole blood sample on the porous spreading layer, and Figure 1B shows progress of the permeation of the sample through the multilayer analytical film wherein a material component (such as erythrocyte, etc.) contained in the sample is filtered off in the porous spreading layer and remains on or in a lower portion of the lower surface of the spreading layer, and a liquid component spreads on the spreading layer and passes through the light-reflecting layer into the reagent layer.

In the figures, the numerals mean the following: 1 transparent support; 2 reagent layer; 3 light-reflecting layer; 4 porous spreading layer; 11 material component; 12 liquid component; 13 whole blood sample; 14 color-formed area; L light source for reflection photometry (not shown); D device for photometry (not shown)

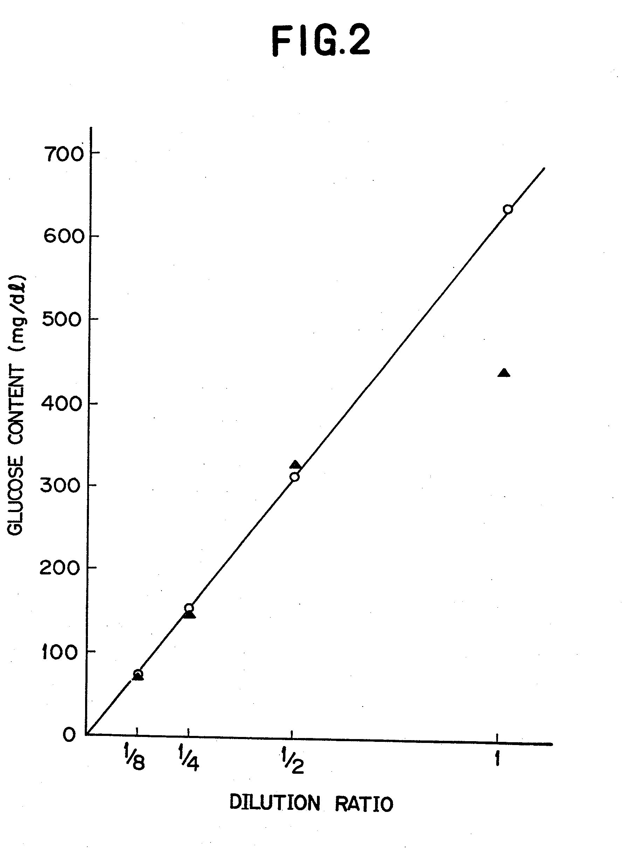

Figure 2 is a graph showing the relationship between a dilution ratio and the measured value of glucose content obtained in Example 2, wherein the marks of vacant circle represent the values determined according to the hexokinase G-6-PDH method and the marks of triangle represent the values determined according to the method using the multilayer analytical film.

## DETAILED DESCRIPTION OF THE INVENTION

The method of firstly diluting a whole blood with an aqueous diluent and then spotting the diluted sample on the porous spreading layer of the multilayer analytical element, which is characteristic feature of the analytical method of the present invention is described in more detail hereinafter.

A stabilizer such as a glycolysis inhibitor or anticoagulant is added to a whole blood collected from a human body or animal except that the blood is subjected to analysis immediately after the collection. Then a dilution treatment using an aqueous diluent is carried out. The aqueous diluent used for carrying out the dilution treatment is a liquid which has substantially no hemolytic action and preferably has such a property that it does not substantially cause change in the fluidity (viscosity) of the whole blood sample diluted under agglutination of erythrocyte. Examples of the aqueous diluents having such a property include isotonic solutions containing an inorganic salt such as physiological saline solution (for example, isotonic sodium chloride solution, Ringer's solution, etc.) and isotonic solutions whose viscosity is adjusted by addition of a water-soluble or water-dispersible organic substance such as dextran, polyvinyl pyrrolidone or albumin in a physiological saline solution. The term "isotonic solution" used herein includes not only aqyeous solutions having isotonic osmotic pressure being critically equal to that of the whole blood, but also those having osmotic pressure being approximate to that of the whole blood, said pressure being in the range in which blood is substantially not hemolyzed. Accordingly, as the physiological saline solution or an organic substance-containing physiological saline solution, there are included not only aqueous solutions having a typical salt content, but also those wherein the salt content or the organic substance content is varied for the purpose of facilitating analytical procedure or accoridng to analytical accuracy. If desired, other conventional additives such as surfactant, antifoaming agent, antiseptic agent, etc. may be incorporated into the aqueous diluent in such an amount that they do not interfere with the analysis to be conducted. As the organic solvents, alcohols such as methanol, ethanol, benzyl alcohol, etc. and other organic liquids may be added to the aqueous diluent.

In the method of the present invention, if a volume (before mixing) of the aqueous diluent to be mixed per a volume of the whole blood sample is termed $a$, said $a$ is generally from 0.1 to 10, preferably 0.5 to 3.0. When a dilution coefficient (d) is defined by $d = (1+a)/1$ and a dilution ratio (1/d) is defined by $1/d = 1/(1+a)$, the value of $a$ should be preferably such a value that d is a lower integer, and 1, 2 or 3 is particularyly preferable for $a$.

The dilution procedure can be done by any of the following method: (1) an aqueous diluent is added to a whole blood; (2) a whole blood is added to an aquous diluent; and (3) A whole blood and an aqueous diluent is introduced in a certain vessel at a substantially same time. It is preferred that after the whole blood and aqueous diluent are combined, they are gently stirred and shaken to thoroughly mix the blood plasma in the whole blood with the aqueous diluent or to bring the mixture into a state wherein the material component in the whole blood is uniformly present throughout the mixture.

For the convenience of the procedure of diluting the whole blood, it is possible to conduct mixing in such a manner, for example, that a certain volume of the aqueous diluent is sucked up by a spotting means such as micropipette and successively a certain volume of the whole blood is sucked up by the same micropipette, thus mixing both components. After conducting the dilution operation in this way, the spotting procedure on the porous spreading layer is carried out in a manner mentioned below. Accordingly, the present invention include a method of the analysis, wherein the dilution of the whole blood is performed by the use of a spotting means capable of producing a liquid drop at the pointed end through pushing out the liquid sample contained therein, in which the diluting process comprises successively drawing up a specified volume of the aqueous diluent and a specified volume of the whole blood into the spotting means and the applying process is performed by pushing out both liquids through the pointed end of the spotting means to spot the liquids on the spreading layer of the multilayer analytical element.

The diluted whole blood is spotted (i.e., added dropwise or deposited) on the porous spreading layer of the multilayer analytical element according to the procedure described in the afore-mentioned patent specifications and literatures, and, if necessary, incubated. The optical density of the color-developed area is measured from the side of the transparent support by a photometric measuring method, and the content of an analyte contained in the whole blood sample is determined according to the principal of colorimetry. It is also possible that the measurement of the color-formed (color-developed) area of the multilayer analytical element is conducted by fluorometry.

The content of the analyte can be optionally determined as the content contained in the whole blood sample after or before dilution. Since the measured value of the optical density in the color-formed area can be directly converted into the analyte content of the diluted whole blood sample, the analyte content of the whole blood sample before dilution can be obtained by multiplying the obtained analyte content of the diluted whole blood sample by the aforementioned dilution coefficient (d). It is understood that the multiplication can be easily made when d is 2, 3 or 4. Thus, the case where a is 1, 2 or 3 is particularly preferred.

Since there is a great difference in the hematocrit value of the whole blood between individuals or individual bodies, the volume ratio of blood plasma as a liquid component in a whole blood varies over a wide range depending on the hematocrit value. In the method of present invention, however, the analyte content of the whole blood sample before dilution can be obtained as a value corresponding to the measured value of the standard quantitative analysis from the analyte content of the diluted whole blood sample merely by using the total volume of the whole blood sample before dilution and the dilution coefficient (d). This is a main characteristic feature of the the method of the present invention.

If desired, there may be used a method wherein the analyte content of the whole blood sample before dilution is determined from that of the diluted whole blood sample by using $d \cdot \varepsilon$ or $(d + \varepsilon)$ as a coefficient from said dilution coefficient $d$ and a complementary coefficient $\varepsilon$. Further, the analyte content of the diluted whole sample can be measured by the method of the present invention, and the measured analyte content per se may be used as the desired value.

It can be decided whether the analyte content, measured by the method of the present invention, of the diluted whole blood sample is used, a value obtained by multiplying said analyte content by d is used, or a value obtained by using $d \cdot \varepsilon$ or multiplying $(d + \varepsilon)$ (wherein $\varepsilon$ is a complementary coefficient) is used, in view of the correlation between the measured value obtained by the method of the present invention and that obtained by the conventional quantitative analysis (which has been used as standard quantitative anlytical method) depending on kinds of analytes.

The following examples further illustrate the present invention in more detail.

## Example 1

Method of quantitatively measuring glucose content with multilayer analytical film for glucose analysis by diluting whole blood with diluent

A fresh blood collected from a human body in the presence of heparin was centrifuged to separate it into a blood plasma component and a blood cell component. Then both components were mixed together by changing the ratio of them to re-prepare a whole blood sample in such manner that the hematocrit value was adjusted to a value within the range of from 40 to 70 %. Glucose was added to and dissolved in each of the whole bloods in such an amount as to give glucose content of 100, 200, 300. 400 and 500 mg/dl, respectively, to prepare 12 kinds of whole blood samples having different hematocrit values and glucose content set forth in Table 1.

A stock solution for an aqueous diluent having the following composition was prepared.

Composition of stock solution for aqueous diluent

| | |
|---|---|
| NaCl | 90 g. |
| $Na_2HPO_4$ | 13.65 g. |
| $NaH_2PO_4 \cdot 2H_2O$ | 2.46 g. |
| Water | amount to make the total amount 100 ml. |

When used, this stock solution was diluted 10 times as much as the original volume to prepare an aqueous diluent (pH = 7.4).

100 µl of the aqueous diluent was added to 100 µl of each of the above whole blood samples to dilute the whole blood sample exactly twice as much as the volume of the sample (dilution coefficient $d$ : 2.00). Thus, 12 kinds of whole blood samples diluted twice as much as the

previously prepared whole blood sample were prepared.

A reagent layer for quantitative analysis of glucose content in blood was coated on the surface of a transparent polyethylene terephthalate (PET) film having a gelatin subbing layer and a thickness of 185 μm in such an amount as to give a dry layer of 15 μm thick and dried.

The reagent layer had the following composition.

| | |
|---|---|
| Peroxidase | 25,000 IU |
| 1,7-Dihydroxynaphthalene | 5 g. |
| 4-Amino-2,3-dimethyl-1-(2,4,6-trichlorophenyl)-3-pyrazoline-5-one | 18 g. |
| Gelatin | 200 g. |
| Nonion HS 210 (surfactant manufactured by Nippon Oil and Fat Co., Ltd. Japan: polyoxyethylene nonylphenyl ether) | 2 g. |

The surface of the reagent layer was then coated with a light-blocking layer in such an amount as to give a dry layer of 15 μm thick, and dried, said light-blocking layer being obtained by dispersing 5 g. of fine titanium dioxide powder containing 0.2 g. of Nonionic HS 210 and 50,000 IU of glucose oxidase in 1 g. of gelatin.

4 g. of gelatin was dissolved in 100 ml of water, and 0.2 g. of Nonion HS 210 was added thereto to prepare a coating solution for an adhesive layer. The surface of the light-blocking layer was coated with said coating solution in such an amount as to give a dry layer of 4 μm thick and dired to form an adhesive layer. The adhesive layer was then wetted by supplying thereonto water as a wetting water at 30 g/m$^2$. Then a 100 % cotton broadcloth (No. 100 count cotton broadcloth) was laminated thereon by pressing. The resulting laminated element was dried to give a porous spreading layer, thus obtaining a multilayer analytical element (multilayer analytical film) for quantitative analysis of glucose content.

This material was cut into chips (1.5 cm x 1.5 cm square), and encased in a plastics mount to obtain a chemical analytical slide for quantitative analysis of glucose.

The glucose content of each of the afore-mentioned 12 whole blood samples was determined according to hexokinase G-6-PDH method. The results are set forth in the second column of the left side of Table 1.

6 µl of each of 12 whole blood samples and the 12 whole blood samples diluted twice as much as the original amount was collected by a micropipette, spotted on the spreading layer of the above analytical slide and incubated at 37°C for 6 minutes. Glucose content was determined by reflection photometry from the PET film side and then by colorimetry. The results are set forth in the third and fourth columns from the left side of Table 1. The glucose content of the diluted whole blood sample is expressed as that of the undiluted whole blood by multiplying dilution coefficient (d = 2.00) and set forth in the most right side of Table 1.

It is apparent from the results set forth in Table 1 that when the whole blood samples having a high hematocrit value are used, remarkably low glucose contents are given by the conventioal measuring method comprising determining glucose content by spotting the undiluted whole blood sample on the multilayer analytical element, as compaered with the glucose contents obtained by the hexokinase G-6-PDH method which is authorized as a standard method for glucose content determinatio method, while when the measuring method of the present invention comprising determining glucose content by spotting the diluted whole blood sample on the multilayer analytical element is conducted, there can be obtained measured values well corresponding to those obtained by the hexokinase G-60-DPH method for the whole blood samples having hematocrit values over a wide range of from a normal to a high hematocrit value.

Table 1

| Hematocrit value of whole blood sample (%) | Glucose content of whole blood sample by hexokinase G-6-PDH method (mg/dl) | Glucose content by chemical analytical slide (mg/dl) | | |
| --- | --- | --- | --- | --- |
| | | value of whole blood sample (X) | value(A) of the diluted whole blood sample | value of A x d (d:2.00) |
| 49 | 171 | 186 | 87 | 174 |
| 50 | 267 | 286 | 138 | 276 |
| 49 | 417 | 464 | 211 | 412 |
| 46 | 645 | 578 | 333 | 666 |
| 60 | 166 | 175 | 86 | 172 |
| 60 | 264 | 259 | 133 | 266 |
| 60 | 389 | 334 | 190 | 380 |
| 58 | 615 | .356 | 304 | 608 |
| 72 | 170 | 147 | 82 | 164 |
| 70 | 265 | 233 | 123 | 246 |
| 71 | 399 | 253 | 176 | 352 |
| 65 | 605 | 260 | 298 | 596 |

Remark: Value (X) is a value obtained in accordance with the conventional measuring method, and Value (A) and value (A x d) both are values obtained in accordance with the measuring method of the presnent invention.

## Example 2

The same aqueous diluent (a solution obtained by diluting the stock solution with water 10 times as much as the original volume of the stock solution) as that of Example 1 was added to a whole blood sample having a hematocrit value of 60 % and a glucose content of 645 mg/dl as measured by the hexokinase G-6-PDH method, prepared in a similar manner to that of Example 1, to dilute the whole blood sample exactly twice, four times and eight times as much as the volume of the sample (dilution coefficient d = 2.00, 4.00 and 8.00, respectivley). The diluted whole blood samples were thus prepared. The glucose content of each of undiluted whole blood sample and the above three kinds of the diluted whole blood samples was measured by using the chemical analytical slide for quantitative analysis of glucose content and the hexokinase G-6-PDH method in a similar manner to that of Example 1. The results are set forth in Table 2.

Table 2

| Dilution coefficient of whole blood | Glucose content (mg/dl) | |
| --- | --- | --- |
| | hexokinase G-6-PDH method | analytical slide method |
| 1.00 (not diluted) | 645 | 447 |
| 2.00 | 318 | 330 |
| 4.00 | 153 | 145 |
| 8.00 | 73 | 70 |

Figure 2 is a graph, prepared based on the measured values of Table 2, wherein the ordinates represents glucose content measured and the abscissas represetns dilution ratio (reciprocal of dilution coefficient). It is confirmed from Table 2 and Figure 2 that the measured values obtained by the hexolinase G-6-PDH method which is authorized as a standard method for the measurement of glucose, lie on a straight line to the dilution ratio including non-dilution case. On the other hand, in the dry analytical method using the multilayer analytical element for quantitative analysis of glucose content, it is confirmed that when dilution coefficient is 1 (not diluted), the measured value greatly deviates from the straight line on which the measured values of the hexokinase G-6-PDH method lie, but when the dilution ratio is within the range of from 1/2 to 1/8, the measured values are distributed on positions standing closely to said straight lien and have a tendency to lie on the straight with a decrease in dilution ratio (namely, with increase in dilution coefficient). It is clear that the above fact shows the effectiveness of the method of the present invention.

- 17 -                                    0123443

CLAIMS:

1.    A method of quantitatively analyzing an analyte contained in a whole blood sample by the use of a multilayer analytical element having at least one reagent layer provided on one side of a light-transmissive water-impermeable support and a porous spreading layer forming the outermost layer of said element and being able to supply a liquid sample applied on the surface of said spreading layer to said reagent layer keeping a substantially constant volume per unit area, the method comprising applying the whole blood sample on the porous spreading layer:
which is characterized in that the whole blood sample is a blood sample prepared by dilution of a whole blood with an aqueous diluent in a specified volume ratio.

2.    The method of analysis as claimed in claim 1, wherein said specified volume ratio is within the range of from 0.1 to 10 volumes of the aqueous diluent per one volume of the whole blood.

3.    The method of analysis as claimed in claim 1, wherein said specified volume ratio is within the range of from 0.5 to 3 volumes of the aqueous diluent per one volume of the whole blood.

4.    The method of analysis as claimed in claim 1, wherein said aqueous diluent does not hemolyze the whole blood.

5.    The method of analysis as claimed in claim 4, wherein said aqueous diluent is a substantially isotonic solution of the whole blood.

6.    The method of analysis as claimed in claim 5, wherein said substantially isotonic solution is a physiological saline solution.

7.    The method of analysis as claimed in claim 5, wherein said substantially isotonic solution is an aqueous solution of a hydrophilic water-soluble or water-dispersable organic substance in a physiological saline solution.

8.    The method of analysis as claimed in claim 5, wherein said substantially isotonic solution is an aqueous solution of a water-miscible organic liquid in a physiological saline solution.

9.    The method of analysis as claimed in claim 6, wherein said organic liquid is at least one compound selected from the group consisting of methanol, ethanol and benzyl alcohol.

10.    The method of analysis as claimed in claim 1, wherein said dilution of the whole blood is performed by adding the aqueous diluent to the whole blood.

11.    The method of analysis as claimed in claim 1, wherein said dilution of the whole blood is performed by adding the whole blood to the aqueous diluent.

12.    The method of analysis as claimed in claim 1, wherein said dilution of the whole blood is performed by introducing the whole blood and the aqueous diluent in a vessel at a substantially same time.

0123443

13. The method of analysis as claimed in claim 1, wherein said dilution of the whole blood is performed by the use of a spotting means capable of producing a liquid drop at the pointed end through pushing out the liquid sample contained therein, in which the diluting process comprises successively drawing up a specified volume of the aqueous diluent and a specified volume of the whole blood into the spotting means and the applying process is performed by pushing out both liquids through the pointed end of the spotting means to spot the liquids on the spreading layer of the multilayer analytical element.

14. The method of analysis as claimed in claim 13, wherein said spotting means is a micropipette.

1/2.

# FIG. I A

# FIG. I B

# FIG.2